# EUROPEAN PATENT APPLICATION

(11) **EP 2 476 666 A1**
(43) Date of publication of application: **18.07.2012**
(21) Application number: 10813814.0
(22) Date of filing: 03.09.2010
(51) Int. Cl.: C07C 303/38, C07C 303/36, C07C 311/48, C07C 311/49

(54) **METHOD FOR PRODUCING BIS(SULFONYL)IMIDE AMMONIUM SALTS, BIS(SULFONYL)IMIDE, AND BIS(SULFONYL)IMIDE LITHIUM SALTS**

(30) Priority: 04.09.2009 JP 2009205223
(71) Applicant: Asahi Glass Company, Limited, Tokyo 100-8405 (JP)
(72) Inventor: SEKI, Ryuji, Tokyo 100-8405 (JP); FURUTA, Shouji, Tokyo 100-8405 (JP); IWAYA, Masao, Tokyo 100-8405 (JP)
(74) Representative: Ehlich, Hendrik
(86) International application number: PCT/JP2010/065164
(87) International publication number: WO 2011/027867

(57) **Abstract**

To provide methods for producing a bis(sulfonyl)imide ammonium salt, a bis(sulfonyl)imide and a bis(sulfonyl)imide lithium salt simply and in good yield. A method for producing a bis(sulfonyl)imide ammonium salt, which comprises reacting a compound of the formula R-CHF-SO₂X (wherein R is a C₁₋₄ fluorinated alkyl group which may contain an etheric oxygen atom, or a fluorine atom, and X is a fluorine atom or a chlorine atom) with ammonia in the absence of a catalyst. Further, methods for producing a bis(sulfonyl)imide and a bis(sulfonyl)imide lithium salt by using the bis(sulfonyl)imide ammonium salt.

## Description

### TECHNICAL FIELD

The present invention relates to methods for producing a bis(sulfonyl)imide ammonium salt, a bis(sulfonyl)imide compound and a bis(sulfonyl)imide lithium salt.

### BACKGROUND ART

A bis(sulfonyl)imide lithium salt having a chain structure has excellent electrochemical properties and is thus useful as an electrolyte for a lithium cell such as a lithium primary battery or a lithium ion secondary battery. As such a bis(sulfonyl)imide lithium salt, a bis(sulfonyl)imide lithium salt having a perfluoroalkyl group such as Li[N(SO₂CF₃)₂] or a bis(sulfonyl)imide lithium salt having a fluoroalkyl group such as Li[N(SO₂-CFH-CF₃)₂] is, for example, known (Patent Document 1).

The following methods are, for example, known as methods for producing such a bis(sulfonyl)imide lithium salt.
(1) A method which comprises reacting Rf¹-SO₂-X¹ with ammonia in the presence of an alkali metal fluoride catalyst (such as potassium fluoride) to obtain a bis(sulfonyl)imide salt (such as a potassium salt), and then reacting the salt with e.g. lithium carbonate to obtain a bis(sulfonyl)imide lithium salt (Patent Document 2). Here, Rf¹ is a C₁₋₁₂ fluoroalkyl group, perfluoroalkyl group, fluoroallyl group or fluoroalkenyl group, and X¹ is fluorine or chlorine.
(2) A method which comprises reacting Rf²-SO₂-F with ammonia in the presence of a tertiary amine catalyst (such as triethylamine) to obtain a bis(sulfonyl)imide salt (such as a triethylammonium salt), and then reacting the salt with lithium hydroxide, lithium hydrogencarbonate or the like to obtain a bis(sulfonyl)imide lithium salt (Patent Document 3). Here, Rf² is a C₁₋₁₂ fluoroalkyl group, perfluoroalkyl group, fluoroallyl group or fluoroalkenyl group.

### PRIOR ART DOCUMENTS

### PATENT DOCUMENTS

Patent Document 1: JP-A-2000-260400
Patent Document 2: JP-A-2001-288193
Patent Document 3: Japanese Patent No. 3,117,369

### DISCLOSURE OF INVENTION

### TECHNICAL PROBLEM

In each of the methods (1) and (2), the catalyst to be used is readily reducible, thus leading to deterioration of a secondary battery if such a catalyst remains in an electrolyte for the battery. Therefore, it is required to sufficiently separate the catalyst. However, if purification such as recrystallization is carried out in order to separate the catalyst, the yield of the bis(sulfonyl)imide lithium salt substantially decreases (by about 40%). Further, there is a problem for disposal of the catalyst, and further, the costs increase by the use of the catalyst.

Further, in the method (2), as a starting material, CF₃SO₂F having a boiling point of lower than 0°C is employed, and at the time of reacting such a starting material compound with ammonia, it is necessary to use a liquefied high pressure gas of ammonia. The production process thereby becomes complex.

It is an object of the present invention to provide methods for producing a bis(sulfonyl)imide ammonium salt which is useful for the production of a bis(sulfonyl)imide lithium salt, and a bis(sulfonyl)imide compound having the ammonium ion in the bis(sulfonyl)imide ammonium salt converted to a proton, simply and in good yield. Further, it is another object of the present invention to provide a method for producing a bis(sulfonyl)imide lithium salt simply and in good yield, by using such a bis(sulfonyl)imide ammonium salt or a bis(sulfonyl)imide compound.

### SOLUTION TO PROBLEM

The present invention provides the following constructions to accomplish the above objects.
[1] A method for producing a bis(sulfonyl)imide ammonium salt, which comprises reacting a compound of the following formula (1) with ammonia in the absence of a catalyst to obtain a bis(sulfonyl)imide ammonium salt of the formula (2):

   R-CHF-SO₂X (1)

   R¹-CHF-SO₂-N(NH₄)-SO₂-CHF-R² (2)

   wherein in the formula (1), R is a C₁₋₄ fluorinated alkyl group which may contain an etheric oxygen atom, or a fluorine atom, and X is a fluorine atom or a chlorine atom; and in the formula (2), each of R¹ and R² which are independent of each other, is a group corresponding to R in the formula (1).
[2] The method for producing a bis(sulfonyl)imide ammonium salt according to the above [1], wherein the compound of the formula (1) is obtained by hydrolyzing a compound of the following formula (3): 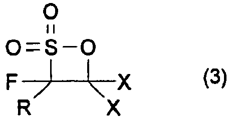 wherein in the formula (3), R is the same as R in the formula (1), and X is the same as X in the formula (1).
[3] The method for producing a bis(sulfonyl)imide ammonium salt according to the above [2], wherein the hydrolysis of the compound of the formula (3) is carried out in the presence of a solvent and a dehalogenating agent.
[4] The method for producing a bis(sulfonyl)imide ammonium salt according to any one of the above [1] to [3], wherein the compound of the formula (1) is CF₃-CHF-SO₂F or CF₂H-SO₂F.
[5] The method for producing a bis(sulfonyl)imide ammonium salt according to any one of the above [1] to [4], wherein the compound of the formula (1) is dissolved in a solvent and then reacted with ammonia.
[6] A method for producing a bis(sulfonyl)imide, which comprises reacting the bis(sulfonyl)imide ammonium salt of the formula (2) obtained by the method as defined in any one of the above [1] to [5], with a Bronsted acid to obtain a bis(sulfonyl)imide of the following formula (4):

   R¹-CHF-SO₂-NH-SO₂-CHF-R² (4)

   wherein in the formula (4), R¹ is the same as R¹ in the formula (2), and R² is the same as R² in the formula (2).
[7] The method for producing a bis(sulfonyl)imide according to the above [6], wherein after the reaction with the Brønsted acid, sublimation for purification is carried out to obtain the compound of the formula (4).
[8] A method for producing a bis(sulfonyl)imide lithium salt, which comprises reacting the bis(sulfonyl)imide ammonium salt of the formula (2) obtained by the method as defined in any one of the above [1] to [5], with at least one lithium salt selected from the group consisting of lithium hydroxide, lithium hydrogencarbonate, lithium oxide and lithium carbonate to obtain a bis(sulfonyl)imide lithium salt of the following formula (5):

   R¹-CHF-SO₂-N(Li)-SO₂-CHF-R² (5)

   wherein in the formula (5), R¹ is the same as R¹ in the formula (2), and R² is the same as R² in the formula (2).
[9] A method for producing a bis(sulfonyl)imide lithium salt, which comprises reacting the bis(sulfonyl)imide compound of the formula (4) obtained by the method as defined in the above [6] or [7], with at least one lithium salt selected from the group consisting of lithium hydroxide, lithium hydrogencarbonate, lithium oxide and lithium carbonate to obtain a bis(sulfonyl)imide lithium salt of the following formula (5):

   R¹-CHF-SO₂-N(Li)-SO₂-CHF-R² (5)

   wherein in the formula (5), R¹ is the same as R¹ in the formula (2), and R² is the same as R² in the formula (2).

### ADVANTAGEOUS EFFECTS OF INVENTION

According to the methods of the present invention, it is possible to produce a bis(sulfonyl)imide ammonium salt and a bis(sulfonyl)imide simply and in good yield. Further, by using such a bis(sulfonyl)imide ammonium salt or a bis(sulfonyl)imide, it is possible to produce a bis(sulfonyl)imide lithium salt simply and in good yield.

### DESCRIPTION OF EMBODIMENTS

In this specification, a compound of the formula (1) will be referred to as a compound (1). Further, compounds of other formulae will be referred to in the same manner.

### [Method for producing bis(sulfonyl)imide ammonium salt]

The method for producing a bis(sulfonyl)imide ammonium salt of the present invention is a method which comprises reacting the following compound (1) with ammonia to obtain the following compound (2) which is a bis(sulfonyl)imide ammonium salt.

R-CHF-SO₂X (1)

R¹-CHF-SO₂-N(NH₄)-SO₂-CHF-R² (2)

In the compound (1), R is a C₁₋₄ fluorinated alkyl group which may contain an etheric oxygen atom (hereinafter referred to as a "fluorinated alkyl group (α)"), or a fluorine atom, and X is a fluorine atom or a chlorine atom. Further, in the compound (2), each of R¹ and R² which are independent of each other, is a group corresponding to R in the compound (1).

The compound (1) is a sulfonyl halide having a R-CHF- group. That is, the compound (1) has a fluoroalkyl group having some of hydrogen atoms of an alkyl group substituted by fluorine atoms, which has at least one hydrogen atom on a carbon atom at the terminal of the bond bonded to the sulfur atom.

R in the compound (1) is a fluorinated alkyl group (α) or a fluorine atom, and it is preferably a fluorinated alkyl group (α), since the reaction to obtain the compound (1) from the after-mentioned compound (3) is thereby easy.

In a case where R is a fluorinated alkyl group (a), the number of its carbon atoms is from 1 to 4. When the number of carbon atoms in the fluorinated alkyl group (α) for R is from 1 to 4, the obtainable compound (4) can be purified by a sublimation method. The number of carbon atoms in the fluorinated alkyl group (α) for R is preferably from 1 to 3, since the purification by a sublimation method of the compound (4) is thereby easier.

Further, in a case where R in the compound (1) is a fluorinated alkyl group (α), the fluorinated alkyl group (α) is preferably such that all of hydrogen atoms in the alkyl group are substituted by fluorine atoms, since the reaction to obtain the compound (1) from the after-mentioned compound (3) is thereby easy.

X in the compound (1) is a fluorine atom or a chlorine atom from the viewpoint of the reactivity of the compound (1) with ammonia. It is preferably a fluorine atom, from the viewpoint of the availability of the starting material.

From the viewpoint of the availability of the starting material, the compound (1) is preferably CF₃-CHF-SO₂F or CF₂H-SO₂F, more preferably CF₃-CHF-SO₂F.

The method of the present invention is characterized by reacting the compound (1) with ammonia in the absence of a catalyst. It is preferred to employ only one type of the compound (1). However, two or more types of the compound (1) may be used in combination.

In the present invention, "in the absence of a catalyst" means that a catalyst is not substantially employed and means that the amount of a catalyst to be used is at most 10 mol%, to 100 mol% of the compound (1). The amount of the catalyst to be used is preferably at most 5 mol%, particularly preferably 0.

As a method for reacting the compound (1) with ammonia, a method may be employed wherein the compound (1) is dissolved in a solvent, and ammonia gas is blown into the solvent. For example, a method may be mentioned wherein a solvent and the compound (1) are introduced into a container such as a flask, and while stirring, ammonia gas is introduced to the gas phase portion or to the liquid phase portion in the container.

The solvent may be a solvent inert to the compound (1) and ammonia and may, for example, be ethyl ether, t-butyl methyl ether, dioxane, tetrahydrofuran, monoglyme, diglyme, triglyme, tetraglyme, diethylene glycol diethyl ether, dichloromethane or carbon tetrachloride.

Blowing of ammonia gas is preferably carried out until absorption of ammonia gas to the solvent becomes no longer observed. The absorption of ammonia gas to the solvent can be confirmed, for example, by analyzing the amount of ammonia gas to be introduced to the gas phase portion and the amount of ammonia gas flowing out from the gas phase portion.

As shown by the following formula, in the reaction, 4 mol of ammonia is reacted with 2 mol of the compound (1) to form 1 mol of the compound (2). Further, as a byproduct, 2 mol of an ammonium halide (ammonium fluoride or ammonium chloride) will be formed. Such an ammonium halide is insoluble and thus can be separated from the compound (2) by filtration.

Further, the reaction of the compound (1) with ammonia may be carried out without using any solvent.

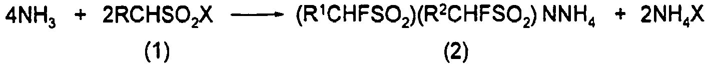

Here, in the compound (2), each of R¹ and R² which are independent of each other, is a group corresponding to R in the compound (1).

The reaction temperature is preferably not higher than the boiling point of the compound (1) in that the reaction can be carried out stably with high efficiency in such a state that the compound (1) is in the form of a solution, and it is more preferably from -20 to 80°C, further preferably from -10 to 40°C. When the reaction temperature is at least -20°C, a sufficient reaction rate is easily obtainable. When the reaction temperature is at most 80°C, the handling efficiency of the compound (1) is improved, and the reaction can be proceeded stably. Further, the reaction time is preferably from 15 minutes to 24 hours.

As a method for producing the compound (1), a method of hydrolyzing the following compound (3) is preferred.

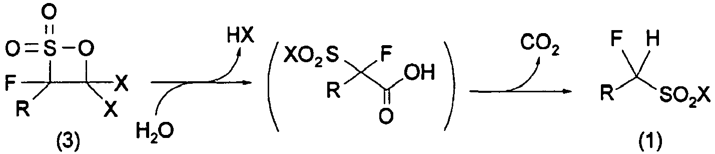

Here, in the compound (3), R is the same as R in the compound (1). Further, in the compound (3), X is the same as X in the compound (1).

By the hydrolysis of the compound (3), the compound (1) is obtained by generating a hydrogen halide, and carbon dioxide by a decarbonation reaction which proceeds simultaneously with the hydrolysis.

The reaction temperature for the hydrolysis of the compound (3) is preferably from -20 to 80°C. Further, the reaction time is preferably from 15 minutes to 24 hours.

The method for hydrolyzing the compound (3) may, for example, be a method of gradually adding distilled water, etc. to a mixture of the compound (3), a solvent and a dehalogenating agent (hereinafter referred to as a "de-HX agent") with stirring, while maintaining the reaction temperature to be within the above range, and continuously withdrawing carbon dioxide which is generated.

The solvent to be used for the hydrolysis of the compound (3) may be the same solvent as the solvent mentioned for the reaction of the compound (1) with ammonia.

As the de-HX agent, a basic compound is preferred, and an alkali metal fluoride, hydride, carbonate, hydrogencarbonate or hydroxide is more preferred. As specific examples, sodium fluoride, sodium hydride, potassium hydride, sodium carbonate, potassium carbonate, cesium carbonate, sodium hydrogencarbonate, potassium fluoride, potassium hydrogencarbonate, sodium hydroxide, potassium hydroxide, cesium hydroxide, etc. may be mentioned.

The amount of the de-HX agent is preferably from 1.0 to 10.0 times by molar ratio to the compound (3).

In the compound (2), each of R¹ and R² which are independent of each other, is a group corresponding to R in the compound (1), and the preferred examples are also the same as R in the compound (1).

In a case where one type of the compound (1) is used alone, the compound (2) is one type of compound wherein R¹ and R² are the same. In a case where two or more types of the compound (1) are used, the compound (2) will be a mixture of ones wherein R¹ and R² are the same and different.

### [Method for producing bis(sulfonyl)imide]

The method for producing a bis(sulfonyl)imide of the present invention is a method which comprises reacting the compound (2) obtained by the above method with a Brønsted acid to obtain the following compound (4) which is a bis(sulfonyl)imide. The Brønsted acid means a substance (proton donor) which presents a proton (H⁺) to the compound (2).

R¹-CHF-SO₂-NH-SO₂-CHF-R² (4)

Here, in the compound (4), R¹ is the same as R¹ in the compound (2). Further, in the compound (4), R² is the same as R² in the compound (2).

The reaction of the compound (2) with the Brønsted acid can be carried out by adding the Brønsted acid directly to the compound (2).

The reaction temperature is preferably from -20 to 80°C. Further, the reaction time is preferably from 15 minutes to 24 hours.

The Brønsted acid may, for example, be sulfuric acid, phosphoric acid or hydrochloric acid.

The amount of the Brønsted acid to be used is preferably from 1.0 to 10.0 times by molar ratio to the compound (2).

The compound (4) is obtained by solvent extraction using an organic solvent such as toluene after the above-mentioned reaction with the Brønsted acid, and concentrating and drying the organic solvent phase.

Further, the compound (4) is sublimed under a reduced pressure of from 10 to 1,000 Pa within a temperature range of from 50 to 200°C. Therefore, after the reaction, its sublimation for purification may be carried out.

### [Method for producing bis(sulfonyl)imide lithium salt]

The method for producing a bis(sulfonyl)imide lithium salt of the present invention is a method of obtaining the following compound (5) by the following method (a) or (b). Such a lithium salt may be prepared by a method of directly converting the ammonium salt to the lithium salt, or by a method via the sulfonimide. However, the method via the sulfonimide is preferred, since a high purity product is thereby readily obtainable.
(a) The compound (2) is reacted with at least one lithium salt selected from the group consisting of lithium hydroxide, lithium hydrogencarbonate, lithium oxide and lithium carbonate (hereinafter referred to as a "lithium salt A").
(b) The compound (4) is reacted with the lithium salt A.

   R¹-CHF-SO₂-N(Li)-SO₂-CHF-R² (5)

Here, in the compound (5), R¹ is the same as R¹ in the compound (2). Further, in the compound (5), R² is the same as R² in the compound (2).

### Method (a):

The compound (2) is dissolved in e.g. distilled water to obtain an aqueous solution, and to the aqueous solution, the lithium salt A is added and stirred. Then, from the reaction solution, water is distilled off to obtain the compound (5).

The reaction temperature for the reaction of the compound (2) with the lithium salt A is preferably from 5 to 95°C. Further, the reaction time is preferably from 0.1 to 10 hours.

As the lithium salt A, lithium hydroxide, lithium hydrogencarbonate or lithium carbonate is preferred. As the lithium salt A, various hydrates (such as lithium hydroxide monohydrate, etc.) may also be used.

The amount of the lithium salt A to be used is preferably from 0.8 to 1.2 times, more preferably from 0.95 to 1.05 times, particularly preferably from 0.98 to 1.02 times, by lithium equivalent ratio to the compound (2). When the amount of the lithium salt A to be used is at least 0.8 time, the compound (5) is readily obtainable. When the amount of the lithium salt A to be used is at most 1.2 times, good yield is readily obtainable in the purification step.

### Method (b):

In the same manner as in the Method (a), the compound (4) is dissolved in e.g. distilled water to obtain an aqueous solution, and to the aqueous solution, the lithium salt A is added and stirred. Then, from the reaction solution, water is distilled off to obtain the compound (5).

The reaction temperature for the reaction of the compound (4) with the lithium salt A is preferably from 5 to 95°C. Further, the reaction time is preferably from 0.1 to 10 hours.

As the lithium salt A to be reacted with the compound (4), lithium hydroxide, lithium hydrogencarbonate or lithium carbonate is preferred. As the lithium salt A, various hydrates (such as lithium hydroxide monohydrate, etc.) may also be used.

The amount of the lithium salt A to be used is preferably from 0.8 to 1.2 times, more preferably from 0.95 to 1.05 times, particularly preferably from 0.98 to 1.02 times, by lithium equivalent ratio to the compound (4). When the amount of the lithium salt A to be used is at least 0.8 time, the compound (5) is easily obtainable. When the amount of the lithium salt A to be used is at most 1.2 times, high yield is readily obtainable in the purification step.

The compound (5) obtainable by the method of the present invention is useful, for example, as an electrolyte for a lithium cell such as a lithium primary battery or a lithium ion secondary battery. As a specific application, an electrolyte for a secondary battery may, for example, be mentioned which comprises a lithium salt containing LiPF₆ or LiBF₄ and the compound (5) and, as a solvent, a cyclic carbonate, a chain carbonate, a cyclic ether, a chain ether, a lactone, a chain ester, a sultone, a sulfone, a hydrofluoroether or a glyme.

According to the method of the present invention as described above, it is possible to produce a bis(sulfonyl)imide lithium salt (compound (5)) in good yield. Such an effect in the present invention is brought about by reacting the compound (1) with ammonia in the absence of a catalyst to produce a bis(sulfonyl)imide ammonium salt (compound (2)). Now, such an effect will be described in detail.

In the conventional method for producing a bis(sulfonyl)imide lithium salt as disclosed in Patent Documents 2 and 3, an alkali metal fluoride catalyst or a tertiary amine catalyst is used as mentioned above. This is because if a sulfonyl halide such as Rf¹-SO₂-X¹ and ammonia are reacted in the absence of a catalyst, the reaction is likely to stop at the time when Rf¹-SO₂-NH₂ is formed. However, in order to prevent deterioration of a secondary battery, it is necessary to sufficiently remove such a catalyst, and if purification such as recrystallization is carried out for that purpose, the yield of the desired bis(sulfonyl) lithium salt substantially decreases.

The present inventors have carried out a study on such a problem, and as a result, have found that in a case where the compound (1) (R-CHF-SO₂-X) is used, even in the absence of a catalyst, the reaction does not stop at R-CHF-SO₂-NH₂, and the compound (2) is obtainable. That is, it has been found that in the case of using, as a starting material, a sulfonyl halide having a fluorinated alkyl group having at least one hydrogen atom bonded to a carbon atom at the terminal of the bond bonded to the sulfur atom, the desired bis(sulfonyl) ammonium salt can be obtained despite not using the catalyst. The reason as to why the reaction can be carried out in the absence of a catalyst by using the compound (1), is considered to be as follows.

The sulfonyl halide having -CF₂- at the terminal of the bond bonded to the sulfur atom (such as CF₃-SO₂-F), has at least two strongly electron-attracting fluorine atoms bonded to a carbon atom bonded to the sulfur atom, in the intermediate product (such as CF₃-SO₂-NH₂) formed by the reaction with one molecule of ammonia. Therefore, the electron density on the nitrogen atom of the compound becomes low, whereby the nucleophilicity decreases, and it is considered that in the absence of a catalyst, a further reaction of the intermediate product with the sulfonyl halide compound will not proceed.

Whereas, when the compound (1) is used, in R-CHF-SO₂-NH₂ (such as CHF₂-SO₂-NH₂) to be formed by the reaction with one molecule of ammonia, one hydrogen atom is bonded to the carbon atom at the terminal of the bond bonded to the sulfur atom. Therefore, the number of fluorine atoms bonded to the carbon atom at the terminal of the bond bonded to the sulfur atom is less, and the electron density on the nitrogen atom is high as compared with the above intermediate product (such as CF₃-SO₂-NH₂) having an equal fluorinated alkyl group. Therefore, it is considered that R-CHF-SO₂-NH₂ has an adequate nucleophilicity, and even in the absence of a catalyst, the compound (1) and ammonia are further reacted, whereby the compound (2) is obtainable. The compound (2) thus produced is free from a catalyst and requires no step of removing a catalyst, and thus, it is free from deterioration in the yield due to separation of the catalyst for purification. Thus, by the method of the present invention, it is possible to obtain the compound (2) in good yield.

Further, by using the compound (2) obtained by such a method, it is possible to produce the compound (4) also in good yield. Further, by using such a compound (2) or a compound (4), it is possible to produce the compound (5) also in good yield.

Further, in the method of the present invention, the compound (1) having a relatively high boiling point as compared with a sulfonyl halide having a perfluoroalkyl group such as CF₃SO₂F (boiling point: -25°C), is used. The reason as to why the boiling point of the compound (1) is relatively high as compared with such a sulfonyl halide is that it has a fluorinated alkyl group to which a hydrogen atom is bonded. For example, the boiling point of CF₃-CHF-SO₂F is 63°C, and the boiling point of CF₂H-SO₂F is 60°C. Therefore, the compound (1) can easily be handled within a temperature range at a level of room temperature, and as the ammonia gas, a liquefied high pressure gas may not be used, and their reaction can easily be carried out.

As described in the foregoing, according to the methods of the present invention, it is possible to produce a bis(sulfonyl)imide ammonium salt (compound (2)) and a bis(sulfonyl)imide (compound (4)) simply and in good yield, and it is thereby possible to produce a bis(sulfonyl)imide lithium salt (compound (5)) simply and in good yield.

### EXAMPLES

Now, the present invention will be described in detail with reference to Examples. However, it should be understood that the present invention is by no means restricted by the following description.

Identification and measurements of purities of compounds obtained in Examples were carried out by ¹⁹F-NMR.

### [PRODUCTION EXAMPLE 1] Production of CF₃-CHF-SO₂F

Into a 1 L Hastelloy autoclave, 230 g (1.0 mol) of the following compound (3-1), 500 mL of dichloromethane and 84 g (2.0 mol) of sodium fluoride as a dehydrofluorinating agent, were charged. On a liquid phase nozzle of the autoclave, a 50 mL stainless steel cylinder having 17.5 g (0.97 mol) of distilled water charged, was set up. As the liquid phase nozzle, a pipe made of a PFA resin (tetrafluoroethylene/perfluoroalkyl vinyl ether copolymer) was used.

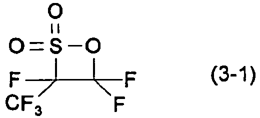

The autoclave was immersed in an ice bath, and distilled water was gradually added, while paying attention so that the internal temperature was maintained to be at most 20°C with stirring, and the reaction was carried out while continuously withdrawing from the gas phase nozzle carbon dioxide generated by a decarbonation reaction which proceeded simultaneously with the hydrolysis.

After completion of the addition of all amount of distilled water, the autoclave was sealed, and the internal temperature was raised to 60°C, and stirring was continued for 2 hours. Then, the autoclave was cooled, and the content was recovered, and the solid content was filtered off, followed by rectification to obtain 169 g (0.92 mol) of CF₃-CHF-SO₂F (compound (1-1)) having a purity of 99%. The yield was 92%.

### [EXAMPLE 1] Production of CF₃-CHF-SO₂-N(NH₄)-SO₂-CHF-CF₃

Into a 300 mL four-necked flask, 50 g (0.27 mol) of the compound (1-1) obtained in Production Example 1 and 50 mL of tetrahydrofuran were charged. The flask was immersed in an ice bath, and ammonia gas was introduced into the gas phase portion, while paying attention so that the internal temperature was maintained to be from 10 to 15°C with stirring. After continuing the introduction of the ammonia gas until absorption of ammonia gas was no longer observed, the formed ammonium fluoride was filtrated, and the filtrate was concentrated and dried to obtain 48.7 g (yield: 88%) of a slightly yellow solid having a ¹⁹F-NMR purity of 89%.

Prior to identification of the desired compound, to make sure, the slightly yellow solid was recrystallized from a toluene/hexane mixed solvent (mixed ratio of 30:70). Then, identification was made by ¹⁹F-NMR, to confirm that desired CF₃-CHF-SO₂-N(NH₄)-SO₂-CHF-CF₃ (compound (2-1)) having a purity of 99% was obtained.

¹⁹F-NMR(282.65MHz, solvent:CD₃CN, standard:CFCl₃)δ(ppm):-72.25 to -72.33, -72.43 to -72.49(CF₃),-190.92 to -191.20,-191.75 to -192.03(CF).

Further, the amount of the compound (2-1) after recrystallization was 34 g (0.094 mol). The yield of this reaction including the recrystallization was 69%. On the other hand, the yield at the stage before the recrystallization was about 88%. In the present invention, recrystallization to separate a catalyst is not essentially required. Accordingly, if no crystallization is carried out, the compound (2-1) can be obtained simply and in good yield.

### [EXAMPLE 2] Production of CF₃-CHF-SO₂-NH-SO₂-CHF-CF₃

To 34 g (0.094 mol) of the compound (2-1) obtained in Example 1, 100 g of concentrated sulfuric acid was added. Thereafter, solvent extraction using 100 mL of toluene was carried out three times. After the extraction, the toluene phase was collected, and the toluene was concentrated, and a white solid thereby obtained was vacuum-dried.

The white solid was heated under reduced pressure at 100°C under 0.67 kPa, whereby sublimation of the desired product was observed in the gas phase portion in the glass container, and therefore, sublimation for purification was carried out. Thereafter, by ¹⁹F-NMR, it was confirmed that CF₃-CHF-SO₂-NH-SO₂-CHF-CF₃ (compound (4-1)) having a purity of at least 99% was obtained. The amount of the obtained compound (4-1) was 30 g (0.085 mol). The yield of this reaction was 90%.

### [EXAMPLE 3] Production of CF₃-CHF-SO₂-N(Li)-SO₂-CHF-CF₃

30 g (0.085 mol) of the compound (4-1) obtained in Example 2 was dissolved in 100 g of distilled water. To this solution, lithium hydroxide and lithium hydrogencarbonate monohydrate were added in an amount of 3.6 g (0.085 mol). Thereafter, the mixture was stirred at room temperature for 5 hours, and then, water was distilled off to obtain 29.2 g (0.083 mol) of white crystals. The yield was 98%, and the purity by ¹⁹F-NMR was at least 99%.

Prior to identification of the desired compound, to make sure, the white crystals were recrystallized from a dioxane/acetonitrile mixed solvent (mixed ratio of 5:1). Thereafter, identification was carried out by ¹⁹F-NMR, whereby it was confirmed that the desired CF₃-CHF-SO₂-N(Li)-SO₂-CHF-CF₃ (compound (5-1)) having a purity of at least 99% was obtained. The amount of the compound (5-1) after the recrystallization was 21 g (0.060 mol). The yield of this reaction was 70%.

In Example 3, prior to the identification by ¹⁹F-NMR, recrystallization was carried out to make sure, but by using the method of the present invention, an operation to separate a catalyst is not required. Therefore, it is possible to obtain a bis(sulfonyl)imide lithium salt in good yield without carrying out recrystallization.

### INDUSTRIAL APPLICABILITY

The compound obtainable by the method of the present invention is useful as an electrolyte for a lithium battery.

The entire disclosure of Japanese Patent Application No. 2009-205223 filed on September 4, 2009 including specification, claims and summary is incorporated herein by reference in its entirety.

## Claims

1. A method for producing a bis(sulfonyl)imide ammonium salt, which comprises reacting a compound of the following formula (1) with ammonia in the absence of a catalyst to obtain a bis(sulfonyl)imide ammonium salt of the formula (2):
R-CHF-SO₂X (1)
R¹-CHF-SO₂-N(NH₄)-SO₂-CHF-R² (2)
wherein in the formula (1), R is a C₁₋₄ fluorinated alkyl group which may contain an etheric oxygen atom, or a fluorine atom, and X is a fluorine atom or a chlorine atom; and in the formula (2), each of R¹ and R² which are independent of each other, is a group corresponding to R in the formula (1).

2. The method for producing a bis(sulfonyl)imide ammonium salt according to Claim 1, wherein the compound of the formula (1) is obtained by hydrolyzing a compound of the following formula (3): 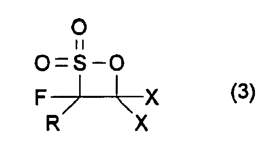 wherein in the formula (3), R is the same as R in the formula (1), and X is the same as X in the formula (1).

3. The method for producing a bis(sulfonyl)imide ammonium salt according to Claim 2, wherein the hydrolysis of the compound of the formula (3) is carried out in the presence of a solvent and a dehalogenating agent.

4. The method for producing a bis(sulfonyl)imide ammonium salt according to any one of Claims 1 to 3, wherein the compound of the formula (1) is CF₃-CHF-SO₂F or CF₂H-SO₂F.

5. The method for producing a bis(sulfonyl)imide ammonium salt according to any one of Claims 1 to 4, wherein the compound of the formula (1) is dissolved in a solvent and then reacted with ammonia.

6. A method for producing a bis(sulfonyl)imide, which comprises reacting the bis(sulfonyl)imide ammonium salt of the formula (2) obtained by the method as defined in any one of Claims 1 to 5, with a Bronsted acid to obtain a bis(sulfonyl)imide of the following formula (4):
R¹-CHF-SO₂-NH-SO₂-CHF-R² (4)
wherein in the formula (4), R¹ is the same as R¹ in the formula (2), and R² is the same as R² in the formula (2).

7. The method for producing a bis(sulfonyl)imide according to Claim 6, wherein after the reaction with the Brønsted acid, sublimation for purification is carried out to obtain the compound of the formula (4).

8. A method for producing a bis(sulfonyl)imide lithium salt, which comprises reacting the bis(sulfonyl)imide ammonium salt of the formula (2) obtained by the method as defined in any one of Claims 1 to 5, with at least one lithium salt selected from the group consisting of lithium hydroxide, lithium hydrogencarbonate, lithium oxide and lithium carbonate to obtain a bis(sulfonyl)imide lithium salt of the following formula (5):
R¹-CHF-SO₂-N(Li)-SO₂-CHF-R² (5)
wherein in the formula (5), R¹ is the same as R¹ in the formula (2), and R² is the same as R² in the formula (2).

9. A method for producing a bis(sulfonyl)imide lithium salt, which comprises reacting the bis(sulfonyl)imide compound of the formula (4) obtained by the method as defined in Claim 6 or 7, with at least one lithium salt selected from the group consisting of lithium hydroxide, lithium hydrogencarbonate, lithium oxide and lithium carbonate to obtain a bis(sulfonyl)imide lithium salt of the following formula (5):
R¹-CHF-SO₂-N(Li)-SO₂-CHF-R² (5)
wherein in the formula (5), R¹ is the same as R¹ in the formula (2), and R² is the same as R² in the formula (2).
